# EUROPEAN PATENT SPECIFICATION

(11) **EP 1 545 244 B1**
(45) Date of publication and mention of the grant of the patent: **20.06.2007**
(21) Application number: 03753498.9
(22) Date of filing: 02.10.2003
(51) Int. Cl.: A23L 1/303, A23J 3/34, A23L 1/275, A23L 1/30

(54) **MODIFIED LUPIN PROTEINS FOR THE PREPARATION OF WATER DISPERSIBLE PRODUCT FORMS OF FAT SOLUBLE COMPOUNDS**
MODIFIZIERTE LUPINPROTEINE ZUR HERSTELLUNG EINER WASSER-DISPERGIERBAREN PRODUKTFORM FETTLÖSLICHER STOFFE
PROTEINES DU LUPIN MODIFIEES POUR LA PREPARATION DE PRODUITS SE DISPERSANT DANS L'EAU DE COMPOSES SOLUBLES DANS LA GRAISSE

(30) Priority: 04.10.2002 EP 02022479
(43) Date of publication of application: 29.06.2005
(73) Proprietor: DSM IP Assets B.V., 6411 TE Heerlen (NL)
(72) Inventor: FUNDA, Elger, 79639 Grenzach-Wyhlen (DE); LEUENBERGER, Bruno, CH-4123 Allschwil (CH); BECK, Markus, 79540 Loerrach (DE); SCHAEFER, Christian, 79618 Rheinfelden (DE)
(74) Representative: Steck, Melanie
(86) International application number: PCT/EP2003/010974
(87) International publication number: WO 2004/030472

(56) References cited:
- EP-A- 0 347 751
- EP-A- 0 859 553
- EP-A- 0 937 412
- EP-A- 1 106 174
- EP-A- 1 219 292
- WO-A-02/19836
- WO-A-99/51106
- DE-A- 10 064 434
- US-A- 4 522 743
- US-B1- 6 335 044
- ALAMANOU S ET AL: "EMULSIFYING PROPERTIES OF LUPIN SEED PROTEINS (LUPINUS ALBUS, SSP. GRAECUS)" DEVELOPMENTS IN FOOD SCIENCE, ELSEVIER, AMSTERDAM, NL, vol. 37B, 6 July 1995 (1995-07-06), pages 2129-2138, XP002068534 ISSN: 0167-4501
- RAYMUNDO A ET AL: "OPTIMIZATION OF THE COMPOSITION OF LOW-FAT-OIL-IN-WATER EMULSIONS STABILIZED BY WHITE LUPIN PROTEIN" JOURNAL OF THE AMERICAN OIL CHEMISTS' SOCIETY, AMERICAN OIL CHEMISTS' SOCIETY. CHAMPAIGN, US, vol. 79, no. 8, August 2002 (2002-08), pages 783-790, XP001124618 ISSN: 0003-021X

## Description

The present invention relates to novel lupin proteins, a process for the manufacture thereof and compositions containing finely divided fat soluble actives and/or colorants in a matrix based on the novel lupin proteins and to a process for preparing these compositions. The present invention further relates to the use of the novel compositions of this invention for coloration and /or for enrichment or fortification of food, beverages, animal feeds, cosmetics or drugs. More particularly, the present invention relates to novel compositions comprising an enzymatically and/or physically treated lupin protein and a fat soluble active or colorant, to a process for preparing these compositions and the use of these compositions as a additives for coloration and/or enrichment or fortification for food, beverages, animal feeds, cosmetics or drugs; and to food, beverages, animal feeds, cosmetics or drugs containing such compositions. WO 99/51106 relates to a modified lupin protein having emulsifying properties.

In one aspect, the present invention relates to a process for the manufacture of a modified lupin protein, which comprises enzymatically hydrolysing a lupin protein of native origin. More particularly, the invention relates to a process which comprises adjusting an aqueous solution or suspension of lupin proteins of native origin having a dry mass content of from 0,1 to 20 % to pH 3 to 9, adding 0,01 to 10 % by weight, in relation to the weight of the dry lupin protein, of a protease, incubating the protein solution or suspension at a temperature of from 5 to 70 °C until obtention of a degree of hydrolysis of from 1 bis 30 %, inactivating of the protease, and converting the hydrolysate into a solid form. If required the said protein hydrolysate may be physically fractionated by common methods such as centrifugation and/or ultrafiltration.

The term "fat soluble active" as used herein comprises a fat soluble vitamins or functionally related compounds which can be used for enrichment or fortification of food, beverages, animal feeds, cosmetics or drugs. Examples of such fat soluble vitamins or the vitamins of the groups A, D, E or K or derivatives thereof such as their acetates or their longer chain fatty acid esters. Examples for functionally related compounds are e.g. polyunsaturated fatty acids (PUFAs) or derivatives thereof, triglycerides rich in polyunsaturated fatty acids such as eicosapentaenoic acid (EPA), docosahexaenoic acid (DHA) or γ-linolenic acid (GLA), or coenzyme Q 10 (CoQ 10). Also included are fat soluble sunfilters, such as UV-A and UV-B filters used in sun care and cosmetic preparations.

The term "colorant" as used herein comprises a carotene or structurally related polyene compound which can be used as a colorant for food, beverages, animal feeds, cosmetics or drugs. Examples of such carotenoids are α- or β-carotene, 8'-apo-β-carotenal, 8'-apo-β-carotenoic acid esters such as the ethyl ester, canthaxanthin, astaxanthin, lycopene, lutein, zeaxanthin or crocetin, α- or β-zeacarotene or mixtures thereof. The preferred carotenoid is β-carotene. It is understood that the above named substances of the categories "fat soluble active" and "colorant" can also be used as mixtures within the novel compositions of the present invention.

The term "lupin protein of native origin" refers to proteins which can be isolated from seeds (including prepared or processed seeds) of any variety of the lupin plant ( e.g., L. albus, L. angustifolius or varieties thereof), or from whole flour or defatted products such as shred or white flakes. Lupin protein concentrates and thermically or chemically pretreated lupin products may be used also for the purposes of the invention.

The term "defatted product" means that the lupin proteins have been defatted prior to their treatment according to the processes of the invention. The defatting may be e.g. accomplished according to the methods disclosed in WO 99/17619 and WO 00/54608. In these documents, mechanical treatment of lupin seeds in order to obtain platelet-shaped flakes, indirectly heating the flakes in the absence of water and subsequently extracting lipids therefrom using a solvent, preferably selected from pentane, hexane, heptane and supercritical CO₂, is disclosed. Defatting may be performed, e.g. using polar solvents or other non-polar solvents, e.g. aliphatic C₁-C₆ mono- and polyalcohols, aromatic C₅-C₁₂ mono- and polyalcohols, aliphatic straight chain, branched or cyclic C₁-C₁₂ alkanes, ethylmethylketone, acetone, acetic acid C₁-C₆ alkyl esters, C₁-C₄ alkyl citrate, dichloromethane, C₂-C₆ dialkylethers and supercritical carbondioxide. Specifically preferred amongst aliphatic mono- and dialcohols and alkanes are methanol, ethanol, butan-1-ol, butan-2-ol, propan-1-ol, propan-2-ol, n-propanol, 1,2-propyleneglycol, propane, butane, pentane, hexane, n-octane iso-octane, cyclohexane.

In a preferred embodiment of the invention, the modified lupin proteins are obtained from solutions or suspension of native lupin protein which have a dry mass content of from about 5-15 % by weight. The solution or suspension is adjusted to pH 3-9, preferably pH 4-8, e.g., by the addition of 0.1-2 M acid or base such as hydrochloric acid and sodium hydroxide, respectively and preferably brought to a temperature of from 40-60 °C, especially 50 °C. The protease used may be a protease having endo-activity or exo-activity. Mixtures of endo- and exo-proteases may be used. Proteases for use in the process of the invention are available from the suppliers, Novozymes, Genencor, AB-Enzymes, Gist-Brocades etc. Preferred proteases are those of bacterial or fungal origin, e.g. from Bacillus licheniformes or Aspergillus oryzae. Enzyme preparations such as Alcalase or Flavourzyme as obtainablke from the firm Novozymes may be used. The protease is added to a provide a concentration of about 0.01- 10 % by weight, preferably about 0.1 - 1% by weight in relation to the weight of the lupin protein. The enzymatic hydrolysis is carried out until a degree of hydrolysis of about 1-30 %, preferably 1-10 % is reached. The degree of hydrolysis can be determined in a manner known to those skilled in the art, e.g. as described by Petersen, D., Nielsen, P.M., Cambmann C., Determination of the Degree of Hydrolysis (DH) based on OPA Reaction, ED-9512723, Novo Nordisk A/S, Dec. 1995; Frister, H., Meisel, H., Schlimme, E., OPA method modified by use of N,N-dimethyl-2-mercaptoethylammonium chloride as thiol component, Fresenius J. Anal. Chem. 330 (1988) 631. The expression "degree of hydrolysis" means the number of hydrolyzed peptide bonds in relation to the total sum of peptide bonds in the lupin protein originally present in the mixture.

The protease is then inactivated, suitably by heating of the incubation batch. In a preferred embodiment of the process of the invention, the enzyme is added in one single step. The enzymatic hydrolysis may also be carried out stepwise. For instance, the protease or a mixture of proteases is added to the incubation batch in an amount of e.g., 1 % whereupon, e.g., after 5-10 minutes (at a temperature of 50°C) further protease or mixture of proteases which may by the same or different from the first added protease or mixture of proteases is added, e.g. in an amount of 2 % whereupon the incubation batch is hydrolyzed at 50 ° for 10 minutes. Using this procedure, starting proteins having a degree of hydrolysis of approximately zero can be used.

Basically, all the above-mentioned parameters are variable. Thus, the temperature of the hydrolysis step may be, in general, 5-70 °C with temperature of 40-60 °C, especially 50° C being preferred. The duration of hydrolysis may vary between 1 and 300 minutes and is preferably 5-10 minutes. The amount of enzyme added may vary between about 0,01-10 % by weight. When the hydrolysis is carried out in a two-step procedure, the amount of enzyme used in the first step is preferably 1 % by weight and in the second step 2 % by weight (based on the dry mass of protein).

The inactivation of the protease can be effected by heat denaturation, e.g., by heating to 80-85 °C for 5-10 minutes. Subsequently, the product may be submitted to fractionating, e.g., by centrifugation or ultrafiltration to separate and isolate the modified lupin protein.

The so-obtained solution or suspension of the modified lupin protein is then converted into a solid form, e.g. a dry powder, e.g., by spray-drying or freeze-drying.

Prior to drying, the modified lupin protein fractions may be submitted to heat treatment (Pasteurisation) e.g. by heating to 80-85 °C for 5-10 minutes to stabilize the product against microbial deterioration.

In still another aspect the present invention relates to modified lupin proteins which are obtainable by the process of the present invention, and to compositions comprising such modified lupin proteins and a fat-soluble active ingredient or colorant, and, optionally, adjuvants and/or excipients. The modified lupin protein is a water-soluble or dispersible polypeptide exerting a protective role towards the inner phase of the dispersion i.e. the fat soluble active or colorant and, therefore, acts as a protective hydrocolloid As a rule the hydrocolloid features surface activity and imparts viscosity, by virtue of which it stabilizes emulsions or suspensions in which it is present with the fat soluble active or colorant.

The compositions of the present invention are preferably solid, powdered compositions wherein the contained fat soluble active or colorant is finely dispersed in a matrix or carrier. The matrix or carrier contains or consists of modified lupin protein as a protective hydrocolloid and, optionally, one or more water soluble excipients and/or adjuvants conventionally used for the formulation of fat soluble actives or colorants.

In the compositions of the present invention, the amount of modified lupin protein is from about 0.5 to about 60.0 wt.-% and the amount of fat soluble active and/or colorant is from about 0.1 to about 80.0 wt.-% based on the total weight of the composition, all components totaling 100 %.

Suitably, the compositions of the present invention (further) contain one or more excipients and/or adjuvants selected from one or more each of a mono- di-, oligo- and polysaccharides, glycerol, triglycerides, water-soluble antioxidants and fat-soluble antioxidants. Solid compositions may in addition contain an anti-caking agent, such as silicic acid, and up to about 10 wt.%, as a rule about 2 to 5 wt.%, of water.

Examples of mono- and disaccharides which may be present in the compositions of the present invention are sucrose, invert sugar, glucose, fructose, lactose, maltose, saccharose and sugar alcohols, and examples of the oligo- and polysaccharides are starch and starch hydrolysates, e.g. dextrins and maltodextrins, especially those having the range of 5 - 65 dextrose equivalents (DE), and glucose syrup, especially such having the range of 20 - 95 DE. The term "dextrose equivalent" (DE) denotes the degree of hydrolysation and is a measure of the amount of reducing sugar calculated as D-glucose based on dry weight; the scale is based on native starch having a DE close to 0 and glucose having a DE of 100.

The triglyceride is suitably a vegetable oil or fat, preferably corn oil, sunflower oil, soybean oil, safflower oil, rape seed oil, peanut oil, palm oil, palm kernel oil, cotton seed oil or coconut oil.

The organic solvent may be for example methylene chloride, chloroform, ethyl acetate, dimethyl ether, acetone, ethanol or isopropanol.

The water-soluble antioxidant may be for example ascorbic acid or a salt thereof, preferably sodium ascorbate. The fat-soluble antioxidant may be for example a tocopherol, e.g. dl-α-tocopherol (i.e. synthetic tocopherol), d-α-tocopherol (i.e. natural tocopherol), β- or γ-tocopherol, or a mixture of two or more of these; butylated hydroxytoluene (BHT); butylated hydroxyanisole (BHA); ethoxyquin, propyl gallate; tert. butyl hydroxyquinoline; or an ascorbic acid ester of a fatty acid, preferably ascorbyl palmitate or stearate. Depending on the pH of the aqueous matrix solution the ascorbic acid ester of a fatty acid, particularly ascorbyl palmitate or stearate, may alternatively be added to the water phase.

The compositions of the present invention may be solid compositions, i.e. stable, water-soluble or -dispersible powders, or they may be liquid compositions, i.e. aqueous colloidal solutions or oil-in-water dispersions of the aforementioned powders. The stabilized oil-in-water dispersions, which may be oil-in-water emulsions or may feature a mixture of suspended, i.e. solid, particles and emulsified, i.e. liquid, droplets, may be prepared by the methods already described above.

Typically, a powder composition according to the present invention comprises
- about 0.5 to about 60 wt.-% , preferably about 5 to about 50 wt.-% of a modified lupin protein;
- about 0.1 to about 80 wt.-% preferably about 0.5 to about 60 wt.-% of a fat soluble active and/or a colorant;
- 0 to about 70 wt.-% preferably about 0 to about 40 wt.-% of a mono- or disaccharide;
- 0 to about 70 wt.-% preferably about 0 to about 40 wt.-% of a starch hydrolysate;
- 0 to about 20 wt.-% preferably about 0 to about 10 wt.-% of glycerol;
- 0 to about 50 wt.-% preferably about 0 to about 30 wt.-% of a triglyceride;
- 0 to about 5 % preferably about 0 to about 2 wt.-% of one or more water-soluble antioxidant(s);
- 0 to about 5 % preferably about 0 to about 2 wt.-% of one or more fat-soluble antioxidant(s);
- 0 to about 50 wt.-% preferably about 0 to about 35 wt.-% of a starch;
- 0 to about 5 wt.% preferably about 1 wt.-% of silicic acid; and
- 0 to about 10 wt.-% preferably about 1 to about 5 wt.-% of water;
the percentages of all ingredients totaling 100.

The novel compositions of this invention can be used for coloration and /or for enrichment or fortification for food, beverages, animal feeds, cosmetics or drugs.

By the present invention there are preferably provided compositions comprising β-carotene as a colouring agent. These compositions, when dissolved, dispersed or diluted in/with water to a final β-carotene concentration of 10 ppm are typically characterized by ultraviolet/visible-spectroscopy using deionized water as reference. At a sample thickness of 1 cm the dispersions show an extinction of at least 0.6 (preferably above 1.0) absorbance units at the wavelength of maximum optical density in the range of 400 to 600 nm. This is equivalent to a formal extinction coefficient of β-carotene in aqueous dispersion E(1%, 1cm) of 600 (preferably >1000).

The manufacture of the compositions of the present invention can be carried out in a manner known per se for the preparation matrix-based compositions of fat soluble actives or colorant compositions for coloration and /or for enrichment or fortification for food, beverages, animal feeds, cosmetics or drugs, e.g. as disclosed in European Patent Publications Nos. 0 285 682, 0 347 751, 0 966 889, 1066 761 and 1 106 174, in the PCT Publication No. WO 98/15195 and in Swiss Patent Application No. 1238/92, the contents of which are incorporated herein by reference.

Thus, the compositions of the present invention can be prepared by a process which comprises homogenizing, in an aqueous or colloidal solution of the modified lupin protein and optionally one or more water-soluble excipients and/or adjuvants, a fat soluble active or colorant or a solution or dispersion of the fat soluble active or colorant and optionally one or more fat-soluble excipients and/or adjuvants, and converting the so-obtained dispersion into a solid composition.

Typically, modified lupin protein and, optional water-soluble excipients and adjuvants are dissolved or dispersed in water. The fat soluble active and/or colorant and, optional fat-soluble excipients and adjuvents are either used as such or dissolved or suspended in a triglyceride and/or an organic solvent. The fat soluble active and/or colorant or the solution or dispersion thereof, respectively, is then added to the aqueous protein solution with stirring and the mixture homogenized using conventional technology. The so-obtained oil-in-water dispersion can, after removal of the organic solvent (if present), be converted into a solid composition, e.g. a dry powder.

A further object of the invention is a process for the preparation of the compositions based on a modified lupin protein as described in more detail above which process comprises homogenizing, in an aqueous solution or colloidal solution of the modified lupin protein and optional water-soluble excipients and adjuvants, a solution or dispersion of the fat soluble active and/or colorant and optional fat-soluble adjuvants and, if required, converting the dispersion obtained into a powder.

For the homogenization conventional technologies, such as high pressure homogenization, high shear emulsification (rotor-stator systems), micronization or wet milling, can be applied. Other techniques used for the preparation of fat soluble active or colorant compositions for coloration and /or for enrichment or fortification for food, beverages, animal feeds, cosmetics or drugs are disclosed in European Patent Publications Nos. 937412 and 1008380 and in US Patent Specification No. 6,093,348, the contents of which are incorporated herein by reference.

The so-obtained dispersion, which is an oil-in-water dispersion, can be converted into a solid composition, e.g. a dry powder, using any conventional technology such as spray drying, spray drying in combination with fluidized bed granulation (the latter technique commonly known as fluidized spray drying or FSD), or by a powder-catch technique whereby sprayed emulsion droplets are caught in a bed of an absorbent, such as starch, and subsequently dried.

Beverages wherein the product forms of the present invention can be used as a colorant or a functional ingredient can be carbonated beverages e.g., flavored seltzer waters, soft drinks or mineral drinks, as well as non-carbonated beverages e.g. flavored waters, fruit juices, fruit punches and concentrated forms of these beverages. They may be based on natural fruit or vegetable juices or on artificial flavors. Also included are alcoholic beverages and instant beverage powders. Besides, sugar containing beverages diet beverages with non-caloric and artificial sweeteners are also included.

Further, dairy products, obtained from natural sources or synthetic, are within the scope of the food products wherein the product forms of the present invention can be used as a colorant or as a functional ingredient. Typical examples of such products are milk drinks, ice cream, cheese, yoghurt and the like. Milk replacing products such as soy milk drinks and tofu products are also comprised within this range of application.

Also included are sweets which contain the product forms of the present invention as a colorant or as a functional ingredient, such as confectionery products, candies, gums, desserts, e.g. ice cream, jellies, puddings, instant pudding powders and the like.

Also included are cereals, snacks, cookies, pasta, soups and sauces, mayonnaise, salad dressings and the like which contain the product forms of the present invention as a colorant or a functional ingredient. Furthermore, fruit preparations used for dairy and cereals are also included.

The final concentration of the active ingredient or the colorant which is added via the product forms of the present invention to the food products may be from about 0.1 to about 500 ppm, particularly from about 1 to about 50 ppm based on the total weight of the food composition and depending on the particular food product to be colored or fortified and the intended grade of coloration or fortification.

The food compositions of this invention are preferably obtained by adding to a food product the active ingredient or the colorant in the form of a composition of this invention. For coloration or fortification of a food or a pharmaceutical product a composition of this invention can be used according to methods per se known for the application of water dispersible solid product forms.

In general the composition may be added either as an aqueous stock solution, a dry powder mix or a pre-blend with other suitable food ingredients according to the specific application. Mixing can be done e.g. using a dry powder blender, a low shear mixer, a high pressure homogenizer or a high shear mixer depending on the formulation of the final application. As will be readily apparent such technicalities are within the skill of the expert.

Pharmaceutical compositions such as tablets or capsules wherein the compositions are used as a colorant are also within the scope of the present invention. The coloration of tablets can be accomplished by adding the product forms in form of a liquid or solid colorant composition separately to the tablet coating mixture or by adding a colorant composition to one of the components of the tablet coating mixture. Colored hard or soft shell capsules can be prepared by incorporating a colorant composition in the aqueous solution of the capsule mass.

Pharmaceutical compositions such as tablets such as chewable tablets, effervescent tablets or filmcoated tablets or capsules such as hard shell capsules wherein the compositions are used as an active ingredient are also within the scope of the present invention. The product forms are typically added as powders to the tableting mixture or filled into the capsules in a manner per se known for the production of capsules.

Animal feed products such as premixes, compound feeds, milk replacers, liquid diets or feed preparations wherein the compositions are either used as a colorant for pigmentation e.g. for egg yolks, table poultry, broilers or aquatic animals or as an active ingredient are also within the scope of the present invention.

Cosmetics, toiletries and derma products i.e. skin and hair care products such as creams, lotions, baths, lipsticks, shampoos, conditioners, sprays or gels wherein the compositions are used as a colorant or as an active ingredient are also within the scope of the present invention.

For the production of liquid and solid product forms such as oil-in-water suspensions, oil-in-water emulsions or powders the hydrocolloids used therein act as multifunctional ingredients. For this purpose these hydrocolloids should show a spectrum of functional properties such as excellent dispersion stabilizing properties, high emulsifying capacity and efficiency (the latter being relevant with respect to the particle size distribution of the inner phase of the dispersion), high solubility and well adapted viscosity in aqueous solution, thermal stability, low susceptibility to pH, ion strength and charge, superior film-forming as well as acceptable gas and water vapour barrier properties in solid product forms.

Native lupin proteins are readily commercially available in the form of flours or protein concentrates such as a product Vitaprot Type LP 60 of J. Rettenmaier & Sons.

The steps used for modification of the lupin protein isolate described above lead to overall improved functional properties of the protein such as better emulsifying properties, generally higher solubility in aqueous solution e.g. lower susceptibility to pH and counter ions as well as better cold water solubility, better temperature resistance, and better film-forming properties. Furthermore the viscosity in aqueous solution is decreased significantly and is now in an acceptable range for the purpose of the current invention.

The following Examples illustrate the invention further

### Example 1

An aqueous suspension of native lupin protein is prepared by aqueous extraction of defatted lupin seed at pH 4-5 and subsequent protein extraction at pH 6-8. The aqueous suspension of native lupin protein has a dry matter content of 4 % by weight the dry matter having a protein content of > 90 %. The pH of the suspension is then adjusted to pH 7 and to a temperature of 50°C. Thereafter, 0,5 % by weight of Alcalase 2.4 L (Novozymes) is added. During the enzymatic hydrolysis the pH is maintained constant by the addition of 1 M NaOH solution and the temperature is maintained at 50 °C. After 5-10 minutesd the protease is inactivated by heating of the suspension to 80 °C for 10 minutes. Then the insoluble fraction is separated and the solution is spray-dried at a product temperature of 70-80 °C.

### Example 2 (comparison example)

An aqueous suspension of native lupin protein is prepared by aqueous extraction of defatted lupin seed at pH 4-5 (the soluble fraction representing the pre-extract) and subsequent protein extraction at pH 6-8. The aqueous suspension of native lupin protein has a dry matter content of 4 % by weight the dry matter having a protein content of > 90 %. The suspension is adjusted to pH 4-5 and the insoluble fraction is separated by centrifugation. The supernatant is combined with the acidic pre-extract and the combined solution is purified by ultrafiltration. Thereby, the soluble protein components of the acidic pre-extract and the soluble proteins of the acidified protein extract in the retentate are obtained. The retentate of the ultrafiltration is spray-dried at a product temperature of 70-80 °C.

### Example 3

A composition comprising modified lupin protein and vitamin A is prepared as follows:

### a) Preparation of (aqueous) solution A:

A dry premix of 34.1 g of modified lupin protein L143T (prepared from L. albus Typ Top according to Example 1 and referred to hereinafter as L143T) and 51.2 g of dried glucose syrup Glucidex IT 47 (Roquette Frères, F-Lestrem) was prepared and subsequently mixed with 120 ml of deionized water in a 1 I rection vessel at room temperature. The mixture was heated to 45°C and stirred for about 15 minutes at 45°C.
The pH was around 6.1.

### b) Preparation of (oil-based) solution B:

15.3 g of crystalline vitamin A acetate, 2.7 g of peanut oil and 1.3 g of dl-α-tocopherol were introduced into a 100 ml Erlenmayer flask. A magnetic stirrer bar was added to the mixture. While stirring the mixture was heated to 65°C within 10 minutes.

### c) Preparation of emulsion from solutions A and B:

Solution A was brought to 50°C and while vigorously stirring solution B was added to solution A within 3 minutes and the dispersion was vigorously stirred for another 20 minutes at 50°C. The emulsion was heated to 60°C and stirred for 5 hours in order to investigate its long term physical stability. After the holding time the mean particle size of the inner phase of the emulsion was 388 nm as measured by photon correlation spectroscopy (Coulter N4 Plus).

### d) Preparation of solid composition from the emulsion:

The emulsion was sprayed into a pre-cooled fluidized bed of corn starch. Excess corn starch was removed by sieving and the powder obtained was dried in an air stream at room temperature for about 2 hours. The powder particle fraction (126.5 g) in the range of 0.16-0.50 mm was collected by sieving and characterized with respect to vitamin A content, surface oil contamination, corn starch content and residual humidity. The powder obtained had a vitamin A content of 263'000 IU/g as measured by HPLC analysis. The surface oil contamination was 0.2 % of the vitamin A content. The powder had a corn starch content of 24.4 % and a residual water content of 6.6 %.

### Example 4

A composition comprising modified lupin protein and vitamin E is prepared as follows:

### a) Preparation of (oil-based) solution A:

26.7 g of dl-α-tocopheryl acetate were introduced into a 100 ml Erlenmayer flask. A magnetic stirrer bar was added and the dl-α-tocopherylacetate was heated to 75°C.

### b) Preparation of (aqueous) solution B:

A dry premix of 40.0 g of modified lupin protein L143T was prepared and subsequently mixed with 135 ml of deionized water in a 1 I rection vessel at room temperature. The mixture was heated to 50°C and stirred for about 15 minutes at 50°C. The pH was around 6.1.

### c) Preparation of emulsion from solutions A and B:

Solution B was brought to 55°C and while vigorously stirring solution A was added to solution B and the emulsion was vigorously stirred for another 30 minutes at 55°C. The emulsion was heated to 60°C and stirred for 5 hours in order to investigate its long term physical stability. After the holding time the mean particle size of the inner phase of the emulsion was 521 nm as measured by photon correlation spectroscopy (Coulter N4 Plus).

### d) Preparation of solid composition from the emulsion:

The emulsion was diluted with deionized water to a final water content of 65 % and then spray dried with a Mobile Minor (NiroA/S). The inlet temperature of the drying air was 220°C the outlet temperature was 110°C. The powder was collected and about 1% of silicon dioxide was added. The tocopheryl acetate content of the powder was 25.6 % as measured by HPLC.

### Example 5

A composition comprising modified lupin protein and β-carotene is prepared as follows:

### a) Preparation of (oil-based) solution A:

8.8 g of corn oil and 1.6 g of dl-α-tocopherol were introduced into a 500 ml Erlenmeyer flask. 18.4 g of crystalline β-carotene were dispersed in 206 ml of trichloro methane and the dispersion obtained was added to the corn oil and the tocopherol. A magnetic stirrer bar was added to the mixture and a reflux condenser was fitted to the Erlenmeyer flask. By gently stirring and, at the same time, heating the mixture to about 65°C a solution was obtained.

### b) Preparation of (aqueous) solution B:

A dry premix of of 48.0 g of lupin protein L140T-2 (prepared from L. angustifolius according to Example 1 and referred to hereinafter as L140T-2 ) and 40.8 g of sucrose and 2.4 g of ascorbyl palmitate was prepared and subsequently mixed with 140 ml of deionized water in a 1 I reaction vessel at room temperature. While stirring the mixture was heated to 35°C and by adding 14 ml of a 1 N sodium hydroxide solution the pH of the mixture was brought to 7.9. Subsequently, the mixture was vigorously stirred for a short time in order to achieve a homogeneous solution.

### c) Preparation of emulsion from solutions A and B:

While vigorously stirring, solution A was added to solution B at 35°C and the dispersion was vigorously stirred for another 30 minutes. The reaction vessel was flushed with nitrogen and the dispersion was heated to 55°C. The stirred dispersion was kept at 55°C for 60 minutes. Residual trichloro methane was removed at 50-55°C using a rotary evaporator. After removing entrapped air bubbles by centrifugation the emulsion was gently stirred at 50-55°C for some minutes and then characterized with respect to particle size of the inner phase. The mean particle size of the inner phase of the emulsion was 148 nm, as measured by photon correlation spectroscopy (Coulter N4 Plus).

### d) Preparation of solid composition from the emulsion:

The emulsion was sprayed into a pre-cooled fluidized bed of corn starch. Excess corn starch was removed by sieving and the powder obtained was dried in an air stream at room temperature for about 2 hours. The powder particle fraction(approx. 138 g) in the range of 0.16-0.63 mm was collected by sieving and characterized with respect to carotenoid content, colour intensity and colour hue in aqueous dispersion, content of corn starch and residual humidity.

The obtained powder had a β-carotene content of 12.8 % (11.5 %), as measured by UV/VIS-spectroscopy (HPLC), a content of 25 % corn starch and a residual water content of 5.9 %. It was dispersed in deionized water and the extinction was measured in a 1 cm quartz precision cell against deionized water. For a 10 ppm dispersion of β-carotene an extinction of 1.130 at a wavelength of 481 nm was calculated [E(1%,1 cm) = 1130]. The colour values L*=81.9, a*=22.6 and b*=31 .6 were measured according to the CIE-system for a 5 ppm dispersion of β-carotene with a Hunterlab Ultrascan Spectrocolorimeter (1 cm, TTRAN). Based upon the values of a* and b* a colour hue angle h*=54.4° at a saturation (chroma value) C*=38.8 can be calculated.

### Example 6

A composition comprising modified lupin protein and β-carotene is prepared as follows:

The procedure according to Example 5 was carried out using modified lupin protein L143T.

The obtained powder had a β-carotene content of 14.2 % (12.4 %), as measured by UV/VIS-spectroscopy (HPLC), a content of 20 % corn starch and a residual water content of 5.5 %. It was dispersed in deionized water and the extinction was measured in a 1 cm quartz precision cell against deionized water. For a 10 ppm dispersion of β-carotene an extinction of 0.892 at a wavelength of 478 nm was calculated [E(1 %, 1 cm) = 892]. The colour values L*=84.1, a*=16.8 and b*=27.4 were measured according to the CIE-system for a 5 ppm dispersion of β-carotene with a Hunterlab Ultrascan Spectrocolorimeter (1 cm, TTRAN). Based upon the values of a* and b* a colour hue angle h*=58.5° at a saturation (chroma value) C*=32.1 can be calculated.

### Example 7

A composition comprising modified lupin protein and β-carotene is prepared as follows:

### a) Preparation of (aqueous) solution A:

60 g of modified lupin protein L143T were dissolved in 400 ml of deionized water at 50°C. A dry premix consisting of 64.5 g of sucrose, 121.8 g of a maltodextrin (DE 20-23) and 3.0 g sodium ascorbate was prepared and added to the aqueous protein solution at 50°C (= solution A).

### b) Preparation of (oil-based) solution B:

46.5 g of a triglyceride (Durkex 500: partly hydrogenated soybean oil) and 0.3 g of dl-α-tocopherol were mixed and heated to 170°C. Subsequently, 3.75 g of β-carotene was suspended in the mixture of triglyceride and tocopherol. By stirring for about 10 minutes a clear solution of β-carotene was obtained (= solution B).

### c) Preparation of emulsion from solutions A and B:

A crude emulsion was prepared by adding solution B to solution A while gently stirring. A fine emulsion was obtained by a four passage high pressure homogenizing treatment of the preemulsion at a pressure of 50/400 bar (LAB 1000 of APV).

### d) Preparation of solid composition from the emulsion:

The emulsion was spray dried in a laboratory spray dryer (Mobile Minor of Niro) at an inlet temperature of 190°C and an outlet temperature of 83°C. A fine powder was obtained with a residual water content of 1.8 %. The β-carotene content of the powder was 1.3 % as measured by UV/VIS-spectroscopy and HPLC. The powder was dispersed in deionized water and the extinction was measured in a 1 cm quartz precision cell against water. For a 10 ppm dispersion of b-carotene an extinction of 1.895 at a wavelength of 463 nm was calculated [E(1%, cm) = 1895]. The colour values L*=85.0, a*=9.0 and b*=63.9 were measured according to the CIE-system for a 5 ppm dispersion of b-carotene with a Hunterlab Ultrascan Spectrocolorimeter (1 cm, TTRAN). Based upon the values of a* and b* a colour hue angle h*=82.0° at a saturation (chroma value) C*=64.5 can be calculated.

### Example 8

A Soft Drink formulation with 6 ppm β-carotene can be prepared as follows:

### Formula

| Ingredients | [g] |
|---|---|
| Sugar syrup 64°Brix | 156.2 |
| Na-benzoate | 0.2 |
| Ascorbic acid | 0.2 |
| Citric acid (as aqueous solution 50% w/w) | 5.0 |
| Flavor *) | 0.5 |
| β-carotene 10% as a 1% stock solution **) | 6.0 |
| Water | to 1000.0 |

| | |
|---|---|
| *) Apricot Flavour No. 78511-76 of Givaudan **) i.e., a 1 % aqueous solution (dispersion) of formulations as obtained e.g. in | |

### Example 3 or 4, based on a β-carotene content of 10 % in said formulation

### Procedure:

Dissolve Na-benzoate in part of the water, add sugar syrup, ascorbic acid, citric acid and flavor and finally the β-carotene stocksolution. Dilute the bottling syrup to one liter of beverage.

The β-carotene provides a vivid orange color of a high clarity and with a high coloring strength. Longtime stability tests of three months at ambient conditions showed a good physical stability of β-carotene in beverages and a good color stability in terms of color values L*C*h*.

## Claims

1. Process for the manufacture of a modified lupin protein, which comprises adjusting an aqueous solution or suspension of lupin proteins of native origin having a dry mass content of from 0,1 to 20 % to pH 3 to 9, adding 0,01 to 10 % by weight of a protease, in relation to the dry weight of the lupin protein, incubating the protein solution or suspension at a temperature of from 5 to 70 °C until a degree of hydrolysis of from 1 to 30 % is obtained, and inactivating the protease.

2. A process according to claim 1, wherein the pH of the solution or suspension is adjusted to pH 4 to 8, the amount of protease added is 0,1 to 1 %, and the enzymatic hydrolysis is effected at 20 to 55 °C.

3. A process according to claims 1 or 2, wherein the enzymatic hydrolysis is effected to a degree of hydrolysis of from 1 to 10 %.

4. A process according to any one of claims 1 to 3, wherein a protease having endo-activity is used.

5. A process according to any one of claims 1 to 3, wherein a protease having exo-activity is used.

6. A process according to any one of claims 1 to 3, wherein a protease from bacteria or fungi is used.

7. A process according to any of the preceding claims, wherein the modified lupin protein, after the protease has been inactivated, is brought into a solid form by centrifugation or ultrafiltration, optionally after a fractionation step.

8. Modified lupin proteins obtainable according to the process of any one of claim 1-7.

9. Composition, comprising a modified lupin protein according to claim 8, and a fat-soluble active ingredient or colorant, and, optionally, adjuvants and/or excipients.

10. A composition as in claim 9, wherein the fat soluble active ingredient or colorant is a carotenoid, a fat soluble vitamin, a triglyceride, an oil soluble UV-A or UV-B filter or a mixture thereof.

11. A composition as in claim 10, wherein the carotenoid is α- or β-carotene, 8'-apo-β-carotenal, 8'-apo-β-carotenoic acid ethyl ester, canthaxanthin, astaxanthin, lycopene, lutein, zeaxanthin or crocetin or mixtures thereof.

12. A composition as in claim 10, wherein the fat soluble vitamin is Vitamin A, D, E or K.

13. A composition as in any one of claims 8-12 wherein at least one of a mono-di-, oligo- or polysaccharide, a triglyceride, a water-soluble anti-oxidant, a fat-soluble anti-oxidant, silicic acid and water is additionally present.

14. A composition as in claim 13 wherein the mono- or disaccharide is saccharose, invert sugar, glucose, fructose, lactose or maltose and the composition optionally contains glycerol in addition.

15. A composition as in claim 13 wherein the polysaccharide is a starch or a starch hydrolysate and /or the triglyceride is a vegetable oil or fat.

16. A composition as in claim 15 wherein the starch hydrolysate is a dextrin or a maltodextrin (in the range of 5-65 dextrose equivalents) or a glucose syrup (in the range of 20-95 dextrose equivalents).

17. A composition as in any one of claims 9-16 wherein the amount of modified lupin protein is from about 0.5 to about 60.0 wt.-% and the amount of fat soluble active ingredient or colorant is from about 0.1 to about 80.0 wt.-%.

18. A process for the preparation of a composition as claimed in any one of claims 9-17 which comprises homogenizing, in an aqueous solution or colloidal solution of the modified lupin protein and optional water-soluble excipients and adjuvants, a solution or dispersion of the fat soluble active and/or colorant and optional fat-soluble adjuvants and, if required, converting the dispersion obtained into a powder.

19. Use of a composition as claimed in any one of claims 9-17 for enrichment, fortification and/or coloration for food, beverages, animal feeds, cosmetics or drugs.

20. Food, beverages, animal feeds, cosmetics or drugs containing a composition as claimed in any one of claims 9-17.

## Patentansprüche

1. Verfahren zur Herstellung eines modifizierten Lupinenproteins, bei dem man eine wäßrige Lösung oder Suspension von Lupinenproteinen nativen Ursprungs mit einem Trockenmassegehalt von 0,1 bis 20% auf pH 3 bis 9 bringt, mit 0,01 bis 10 Gew.-% in bezug auf das Trockengewicht des Lupinenproteins mit einer Protease versetzt, die Proteinlösung oder -suspension bei einer Temperatur von 5 bis 70°C so lange inkubiert, bis man zu einem Hydrolysegrad von 1 bis 30% gelangt, und dann die Protease inaktiviert.

2. Verfahren nach Anspruch 1, wobei der pH-Wert der Lösung oder Suspension auf pH 4 bis 8 eingestellt wird, die zugesetzte Proteasemenge 0,1 bis 1% beträgt und die enzymatische Hydrolyse bei 20 bis 55°C durchgeführt wird.

3. Verfahren nach Anspruch 1 oder 2, wobei die enzymatische Hydrolyse bis zu einem Hydrolysegrad von 1 bis 10% durchgeführt wird.

4. Verfahren nach einem der Ansprüche 1 bis 3, wobei eine Protease mit endo-Aktivität verwendet wird.

5. Verfahren nach einem der Ansprüche 1 bis 3, wobei eine Protease mit exo-Aktivität verwendet wird.

6. Verfahren nach einem der Ansprüche 1 bis 3, wobei eine Protease aus Bakterien oder Pilzen verwendet wird.

7. Verfahren nach einem der vorhergehenden Ansprüche, wobei das modifizierte Lupinenprotein, nachdem die Protease inaktiviert worden ist, durch Zentrifugation oder Ultrafiltration, gegebenenfalls nach einem Fraktionierungsschritt, in feste Form gebracht wird.

8. Modifizierte Lupinenproteine, die nach dem Verfahren von einem der Ansprüche 1-7 erhältlich sind.

9. Zusammensetzung, die ein modifiziertes Lupinenprotein nach Anspruch 8 und einen fettlöslichen Wirkstoff oder Farbstoff sowie gegebenenfalls Hilfsstoffe und/oder Trägerstoffe enthält.

10. Zusammensetzung nach Anspruch 9, wobei es sich bei dem fettlöslichen Wirkstoff oder Farbstoff um ein Carotinoid, ein fettlösliches Vitamin, ein Triglycerid, ein öllösliches UV-A- oder UV-B-Filter oder eine Mischung davon handelt.

11. Zusammensetzung nach Anspruch 10, wobei es sich bei dem Carotinoid um α- oder β-Carotin, 8'-apo-β-Carotinal, 8'-apo-β-Carotinsäureethylester, Canthaxanthin, Astaxanthin, Lycopen, Lutein, Zeaxanthin oder Crocetin oder Mischungen davon handelt.

12. Zusammensetzung nach Anspruch 10, wobei es sich bei dem fettlöslichen Vitamin um Vitamin A, D, E oder K handelt.

13. Zusammensetzung nach einem der Ansprüche 8-12, wobei zusätzlich mindestens ein Stoff aus der Reihe Mono-, Di-, Oligo- oder Polysaccharid, Triglycerid, wasserlösliches Antioxidans, fettlösliches Antioxidans, Kieselsäure und Wasser vorliegt.

14. Zusammensetzung nach Anspruch 13, wobei es sich bei dem Mono- oder Disaccharid um Saccharose, Invertzucker, Glucose, Fructose, Lactose oder Maltose handelt und die Zusammensetzung gegebenenfalls zusätzlich noch Glycerin enthält.

15. Zusammensetzung nach Anspruch 13, wobei es sich bei dem Polysaccharid um eine Stärke oder ein Stärkehydrolysat und/oder bei dem Triglycerid um ein pflanzliches Öl oder Fett handelt.

16. Zusammensetzung nach Anspruch 15, wobei es sich bei dem Stärkehydrolysat um ein Dextrin oder Maltodextrin (im Bereich von 5-65 Dextroseäquivalenten) oder einen Glucosesirup (im Bereich von 20-95 Dextroseäquivalenten) handelt.

17. Zusammensetzung nach einem der Ansprüche 9-16, wobei die Menge an modifiziertem Lupinenprotein von ungefähr 0,5 bis ungefähr 60,0 Gew.-% und die Menge an fettlöslichem Wirkstoff oder Farbstoff von ungefähr 0,1 bis ungefähr 80,0 Gew.-% beträgt.

18. Verfahren zur Herstellung einer Zusammensetzung nach einem der Ansprüche 9-17, bei dem man eine Lösung oder Dispersion des fettlöslichen Wirkstoffs und/oder Farbstoffs und gegebenenfalls fettlöslichen Hilfsstoffs in einer wäßrigen Lösung oder einer kolloidalen Lösung des modifizierten Lupinenproteins sowie gegebenenfalls der wasserlöslichen Trägerstoffe und Hilfsstoffe homogenisiert und, falls erforderlich, die erhaltene Dispersion in ein Pulver umwandelt.

19. Verwendung einer Zusammensetzung nach einem der Ansprüche 9-17 zum Anreichern, Versetzen mit und/oder Färben von Nahrungsmitteln, Getränken, Tierfuttermitteln, Kosmetika oder Arzneimitteln.

20. Nahrungsmittel, Getränke, Tierfuttermittel, Kosmetika oder Arzneimittel, die eine Zusammensetzung nach einem der Ansprüche 9-17 enthalten.

## Revendications

1. Procédé de fabrication d'une protéine de lupin modifiée, qui comprend l'ajustement d'une solution ou d'une suspension aqueuse de protéines de lupin d'origine naturelle dont la teneur en masse sèche varie de 0,1 à 20 % à un pH de 3 à 9, l'ajout de 0,01 à 10 % en poids d'une protéase, par rapport au poids sec de la protéine de lupin, l'incubation de la solution ou de la suspension aqueuse à une température variant de 5 à 70 °C jusqu'à l'obtention d'un degré d'hydrolyse variant de 1 à 30 %, et l'inactivation de la protéase.

2. Procédé selon la revendication 1, dans lequel le pH de la solution ou de la suspension est réglé à un pH de 4 à 8, la quantité de protéase ajoutée varie de 0,1 à 1 % et l'hydrolyse enzymatique est effectuée à une température de 20 à 55 °C.

3. Procédé selon l'une des revendications 1 ou 2, dans lequel l'hydrolyse enzymatique s'effectue à un degré d'hydrolyse de 1 à 10 %.

4. Procédé selon l'une quelconque des revendications 1 à 3, dans lequel on utilise une protéase qui possède une activité endo.

5. Procédé selon l'une quelconque des revendications 1 à 3, dans lequel on utilise une protéase qui possède une activité exo.

6. Procédé selon l'une quelconque des revendications 1 à 3, dans lequel on utilise une protéase d'une bactérie ou d'un champignon.

7. Procédé selon l'une quelconque des revendications précédentes, dans lequel, après l'inactivation de la protéase, la protéine de lupin modifiée est convertie en une forme solide par centrifugation ou ultrafiltration, éventuellement après une étape de fractionnement.

8. Protéines de lupin modifiées que l'on peut obtenir selon le procédé de l'une quelconque des revendications 1 à 7.

9. Composition qui comprend une protéine de lupin selon la revendication 8, un ingrédient ou un colorant actif liposoluble et, éventuellement, des adjuvants et/ou des excipients.

10. Composition selon la revendication 9, dans laquelle l'ingrédient ou le colorant actif liposoluble est un caroténoïde, une vitamine liposoluble, un triglycéride, un filtre UVA ou WB liposoluble ou un mélange de ceux-ci.

11. Composition selon la revendication 10, dans laquelle le caroténoïde est un α- ou un β-carotène, un ester éthylique d'acide 8'-apo-β-caroténoïque, une canthaxanthine, une astaxanthine, un lycopène, une lutéine, une zéaxanthine ou une crocétine ou un mélange de ceux-ci.

12. Composition selon la revendication 10, dans laquelle la vitamine liposoluble est la vitamine A, D, E ou K.

13. Composition selon l'une quelconque des revendications 8 à 12, dans laquelle au moins un parmi un mono-, un di-, un oligo- ou un polysaccharide, un triglycéride, un antioxydant hydrosoluble, un antioxydant liposoluble, l'acide silicique et l'eau est en outre présent.

14. Composition selon la revendication 13, dans laquelle le mono- ou le disaccharide est du saccharose, du sucre inverti, du glucose, du fructose, du lactose ou du maltose et la composition comprend éventuellement en outre du glycérol.

15. Composition selon la revendication 13, dans laquelle le polysaccharide est un amidon ou un hydrolysat d'amidon et/ou le triglycéride est une huile ou un gras végétal.

16. Composition selon la revendication 15, dans laquelle l'hydrolysat d'amidon est une dextrine ou une maltodextrine (dans la plage de 5 à 65 d'équivalents de dextrose) ou un sirop de glucose (dans la plage de 20 à 95 équivalents de dextrose).

17. Composition selon l'une quelconque des revendications 9 à 16, dans laquelle la quantité de protéine de lupin modifiée varie d'environ 0,5 à environ 60,0 % en poids et la quantité d'ingrédient ou de colorant actif liposoluble varie d'environ 0,1 à environ 80,0 % en poids.

18. Procédé de préparation d'une composition selon l'une quelconque des revendications 9 à 17, lequel comprend l'homogénéisation, dans une solution aqueuse ou une solution colloïdale de la protéine de lupin modifiée et des excipients et des adjuvants hydrosolubles éventuels, d'une solution ou d'une dispersion de l'ingrédient et/ou du colorant actif liposoluble et des adjuvants liposolubles éventuels et, s'il y a lieu, la conversion de la dispersion obtenue en poudre.

19. Utilisation de la composition selon l'une quelconque des revendications 9 à 17 pour un enrichissement, une fortification et/ou une coloration pour des aliments, des boissons, des aliments pour animaux, des cosmétiques ou des médicaments.

20. Aliments, boissons, aliments pour animaux, cosmétiques ou médicaments comprenant une composition selon l'une quelconque des revendications 9 à 17.
